# EUROPEAN PATENT APPLICATION

(11) **EP 2 974 752 A1**
(43) Date of publication of application: **20.01.2016**
(21) Application number: 14764880.2
(22) Date of filing: 11.03.2014
(51) Int. Cl.: A61L 27/00

(54) **ADHESIVE BONE FILLER AND ADHESIVE BONE FILLER KIT**

(30) Priority: 13.03.2013 JP 2013050139; 01.10.2013 JP 2013206357
(71) Applicant: National Institute for Materials Science, Tsukuba-shi, Ibaraki 305-0047 (JP)
(72) Inventor: TAGUCHI Tetsushi, Tsukuba-shi Ibaraki 305-0047 (JP)
(74) Representative: Calamita, Roberto
(86) International application number: PCT/JP2014/056368
(87) International publication number: WO 2014/142132

(57) **Abstract**

Provided is an adhesive bone filling agent which adheres to bone tissue and hardens, and the adhesive bone filling agent is prepared by mixing a liquid component composed of a buffer solution comprising a water-soluble biocompatible polymer with a powder component comprising calcium phosphate and an organic acid-based crosslinking agent.

## Description

### Technical Field

The present invention relates to an adhesive bone filling agent and an adhesive bone filling agent kit.

### Background Art

In Japan, aging of population is rapidly progressing at present and the aging rate of population is expected to reach 30% in 2020 (that is, 30 million or more people are aged people). Spinal injury to aged people is disturbance of motility to directly lead the aged people to be bedridden, and the bedridden aged patients experience dementia at a high probability, which leads to long-term hospitalization. The vertebral compression fracture is a particularly highly frequent injury among the spinal injuries, is often affected by osteoporosis, and significantly increases with aging.

In the world, 100 million people are exposed to this risk, and 2 million or more cases of fragility fracture are reported every year in the United States. In the report to determine the prevalence of spinal fracture in Japanese people, 25% people who are at the age of from 70 to 74 years and 43% people who are at the age of from 80 to 84 years are with vertebral fracture or spinal deformity and the values are about two times those in the United States.

Hence, the development of a bone fusiogenic vertebral body reconstruction device to minimize the invasion and to maximize the healing power of the patient himself is an urgent task.

Hitherto, as the treatment method of vertebral compression fracture, spinal fusion to achieve the integration of bone by fixing the spine with a metal screw or rod or vertebroplasty to inject a bone filling agent into the vertebral body and thus to reinforce the bone for the purpose of early pain relief has been performed.

Spinal fusion is greatly invasive and thus has a problem that the physical and economic burdens are great to the aged people. On the other hand, as vertebroplasty, there is balloon kyphoplasty (BKP) to create a void at the vertebral compression fracture site using a silicone balloon and to introduce a bone filling agent into the space (Non Patent Literature 1). An instrument for spine surgery using the BKP technique is disclosed (Patent Literature 1).

Vertebroplasty has become a mainstream surgical method due to a low physical burden to the patient. Although this vertebroplasty is a less invasive surgery, a case has been reported that a methyl methacrylate monomer contained in a bone cement leaks to the outside of the vertebral body to cause a complication such as pulmonary embolism or spinal cord injury in the case of using the bone cement as the bone filling agent (Non Patent Literature 2). Furthermore, it is also a problem that the bone cement is nonabsorbable (Patent Literature 2). Moreover, there is also a problem that the surrounding tissue necrotizes by high heat of polymerization.

In addition, a bone paste is absorbable but has a problem in that it is impossible to achieve strength in a short period of time (Non Patent Literature 3) since it takes a long time, in some case, about a week to harden the bone paste until the bone paste exhibits the maximum strength. Furthermore, there is a problem in that the bone paste does not exhibit adhesive property to the cancellous bone inside of the vertebral body part since most of the bone paste is composed of an inorganic component (Patent Literature 3). In addition, it takes a long time of several hours or longer to harden a calcium phosphate-based bone filling agent, and thus the calcium phosphate-based bone filling agent has a problem of not exhibiting adhesive property to bone tissue immediately after application in addition to a risk of leakage of the filling agent.

### Citation List

### Patent Literature

Patent Literature 1: JP 2012-85804 A
Patent Literature 2: JP 2001-510369 W
Patent Literature 3: JP 10-539819 A

### Non Patent Literature

Non Patent Literature 1: SPINE, 26, 151-156 (2001)
Non Patent Literature 2: Acta Radiologica, 48, 89-95 (2007)
Non Patent Literature 3: Journal of Materials Science Materials in Medicine, 6, 340-347 (1995)

### Summary of Invention

### Technical Problem

An object of the present invention is to provide an adhesive bone filling agent which is highly adhesive to the bone tissue, adheres in a short period of time, and is highly absorbable and an adhesive bone filling agent kit. In addition, another object thereof is to provide an adhesive bone filling agent which exhibits high biological safety and has a bending strength set to a moderate strength (1 MPa or more and 50 MPa or less).

### Solution to Problem

The present inventors have found out that it is possible to produce an adhesive bone filling agent which does not use a monomer and adheres to the bone tissue by preparing a bone filling agent having a binary system of solid and liquid which contains a biopolymer (albumin, gelatin) as the liquid component and an organic acid-based crosslinking agent and calcium phosphate as the solid component.

In addition, it has been found out that it is possible to produce an adhesive bone filling agent which does not use a monomer by preparing a bone filling agent having a binary system of solid and liquid which contains a synthetic polymer as the liquid component and an organic acid-based crosslinking agent and calcium phosphate as the solid component, whereby the present invention has been completed.

The present invention has the following configuration.
(1) An adhesive bone filling agent which adheres to bone tissue and hardens, the adhesive bone filling agent including a combination of a liquid component composed of a buffer solution comprising a water-soluble biocompatible polymer and a powder component comprising calcium phosphate and an organic acid-based crosslinking agent.
(2) The adhesive bone filling agent, in which the water-soluble biocompatible polymer is a water-soluble biocompatible biopolymer or a synthetic polymer.
(3) The adhesive bone filling agent according to (2), in which the biopolymer is albumin or gelatin.
(4) The adhesive bone filling agent according (3), in which the albumin is serum albumin of any one species or two or more species of cattle, swine, horse, and human or recombinant albumin.
(5) The adhesive bone filling agent according to (3), in which the gelatin is gelatin of any one species or two or more species of cattle, swine, fish, and human or recombinant gelatin.
(6) The adhesive bone filling agent according to (2), in which the synthetic polymer is a polyether or water-soluble polymer having an amino group in a side chain or at a terminal.
(7) The adhesive bone filling agent according to (6), in which the polyether is a poly(ethylene glycol) derivative having from 3 to 8 branches.
(8) The adhesive bone filling agent according to (7), in which the poly(ethylene glycol) having from 3 to 8 branches is pentaerythritol tetra-poly(ethylene glycol) ether.
(9) The adhesive bone filling agent according to (6), in which the water-soluble polymer is a polypeptide.
(10) The adhesive bone filling agent according to (9), in which the polypeptide is polylysine.
(11) The adhesive bone filling agent according to any one of (1) to (10), in which the calcium phosphate is one kind or a combination of two or more kinds of α-tricalcium phosphate (α-TCP), α'- tricalcium phosphate (α'-TCP), β-tricalcium phosphate (β-TCP), octa-calcium phosphate (OCP), dicalcium phosphate dibasic (DCPD), tetracalcium phosphate monoxide (TeCP), hydroxyapatite (HAp), and calcium monohydrogen phosphate.
(12) The adhesive bone filling agent according to any one of (1) to (11), in which the calcium phosphate is doped with a metal element.
(13) The adhesive bone filling agent according to (12), in which the metal element is one kind or two or more kinds of zinc, copper, strontium, silver, and titanium.
(14) The adhesive bone filling agent according to any one of (1) to (11), in which the calcium phosphate is particles having a size of from 10 to 1000 nm.
(15) The adhesive bone filling agent according to any one of (1) to (14), in which the organic acid-based crosslinking agent is one kind or a combination of two or more kinds of crosslinking agents obtained by modifying all the carboxyl groups of tartaric acid, malic acid, succinic acid, or citric acid with N-hydroxysuccinimide or N-hydroxysulfosuccinimide.
(16) The adhesive bone filling agent according to any one of (1) to (15), in which an alkali component is added to the liquid component or the powder component.
(17) The adhesive bone filling agent according to any one of (1) to (16), in which P/L of a ratio of the first powder component (P (g)) to the liquid component (L (g)) meets a condition of 1/1 < P/L < 3.5/1.
(18) An adhesive bone filling agent kit including a first container enclosing a liquid component composed of a buffer solution comprising a water-soluble biocompatible polymer, a second container enclosing a powder component comprising calcium phosphate and an organic acid-based crosslinking agent, a third container for producing an adhesive bone filling agent by mixing the powder component with the liquid component, and an injection container for injecting the adhesive bone filling agent into a site to be adhered.

### Advantageous Effects of Invention

The adhesive bone filling agent of the present invention is an adhesive bone filling agent which adheres to bone tissue and hardens and configured to include a combination of a liquid component composed of a buffer solution comprising a water-soluble biocompatible polymer and a powder component comprising calcium phosphate and an organic acid-based crosslinking agent, and thus it is possible to increase the adhesive property to the bone tissue, to shorten the adhering time, and to increase the absorbability of the adhesive bone filling agent. The adhesive bone filling agent exhibits high bone adhesive property to be prevented from leaking from the adhesive part to the outside and thus the risk of a complication can be eliminated. In addition, the adhesive bone filling agent also has an advantage that the hardening temperature thereof is low. In addition, it is possible to set the bending strength of the adhesive bone filling agent to a moderate strength (1 MPa or more and 50 MPa or less).

It is possible to greatly decrease the risk of fracture of the vertebral body bone adjacent to the surgical site by setting the bending strength to a moderate strength. The polymethyl methacrylate-based bone cement is too hard and thus concerned to cause further fracture of the peripheral vertebral body bone. On the other hand, the calcium phosphate-based bone filling agent is fragile and thus concerned to cause the fracture of the reinforced part, but a moderate strength thereof is a great advantage. In addition, the calcium phosphate-based bone filling agent exhibits high bio-safety.

The adhesive bone filling agent kit of the present invention includes a first container enclosing a liquid component, a second container enclosing a powder component, a third container for producing an adhesive bone filling agent by mixing the powder component with the liquid component, and an injection container for injecting the adhesive bone filling agent into a site to be adhered, and the liquid component is composed of a buffer solution comprising a water-soluble biocompatible polymer, the powder component is a mixed powder of calcium phosphate of the first powder component and an organic acid-based crosslinking agent of the second powder component, and the adhesive bone filling agent has a configuration that is the adhesive bone filling agent described above, and thus it is possible to easily and quickly perform adhesive treatment without requiring an instrument or a reagent other than this.

### Brief Description of Drawings

Fig. 1 is a schematic view illustrating an example of an adhesive bone filling agent kit of an embodiment of the present invention.
Figs. 2(a) to 2(c) are process views illustrating an example of a method to use an adhesive bone filling agent kit of an embodiment of the present invention.
Fig. 3 is a process view illustrating an example of a method to use an adhesive bone filling agent kit of an embodiment of the present invention.
Fig. 4 is an explanatory view illustrating an example of a crosslinking reaction when tetraamine-terminated poly(ethylene glycol) (TAPEG) is mixed with trisuccinimidyl citrate (TSC) of an organic acid-based crosslinking agent.
Fig. 5 is visual images (photographs) of molded samples after leaving to stand for 24 hours in a wet environment at 37°C.
Fig. 6 is visual images (photographs) of molded samples after preparation.
Figs. 7(a) to 7(c) are process views of a bone adhesive property test.
Fig. 8 is an electron micrograph illustrating an example of the surface of a bone mimetic material of a living body used in the present test.
Fig. 9 is a photograph taken when the adhesive bone filling agent (Test Example A1) is discharged from a syringe.
Fig. 10 is an outline view illustrating the outline of a bending test.
Fig. 11 is a graph illustrating bending stresses of a hardened product of a bone filling agent according to an adhesive bone filling agent of the present invention and BioPex.
Fig. 12 is a graph illustrating the TAPEG concentration dependence of the bending stress of a hardened product of a bone filling agent according to an adhesive bone filling agent of the present invention.
Fig. 13 is a graph illustrating the TSC concentration dependence of the bending stress of the hardened product of a bone filling agent according to an adhesive bone filling agent of the present invention.
Fig. 14 is a graph illustrating the P/L ratio dependence of the bending stress of the hardened product of a bone filling agent according to an adhesive bone filling agent of the present invention.
Fig. 15 is a graph illustrating the α-TCP content dependence of the bending stress of the hardened product of a bone filling agent according to an adhesive bone filling agent of the present invention.
Fig. 16 is a graph illustrating the TAPEG concentration dependence of the bending stress of the hardened product of a bone filling agent according to an adhesive bone filling agent of the present invention.

### Description of Embodiments

### (Embodiments of the present invention)

Hereinafter, the adhesive bone filling agent and the adhesive bone filling agent kit of embodiments of the present invention will be described with reference to the accompanying drawings.

### <Adhesive bone filling agent kit>

First, the adhesive bone filling agent kit of an embodiment of the present invention will be described.

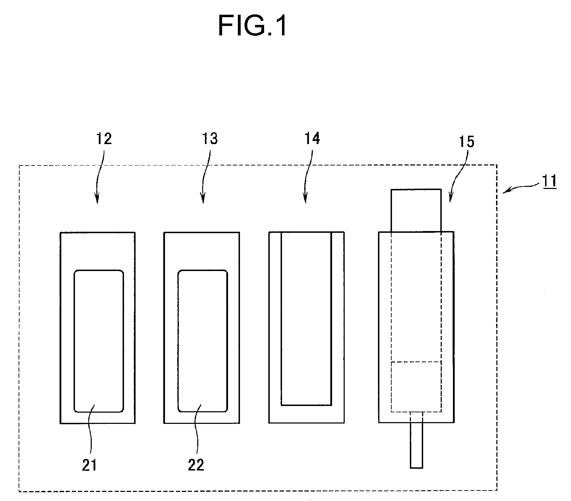

Fig. 1 is a schematic view illustrating an example of the adhesive bone filling agent kit of an embodiment of the present invention.

As illustrated in Fig. 1, an adhesive bone filling agent kit 11 of an embodiment of the present invention is schematically configured to include a first container 12 enclosing a liquid component 21, a second container 13 enclosing a powder component 22, a third container 14 for producing an adhesive bone filling agent by mixing the powder component 22 with the liquid component 21, and an injection container 15 for injecting the adhesive bone filling agent into a site to be adhered by the adhesive agent.

Here, the liquid component 21 is composed of a buffer solution comprising a water-soluble biocompatible polymer, the powder component 22 is a mixed powder of calcium phosphate of the first powder component and an organic acid-based crosslinking agent of the second powder component, and the adhesive bone filling agent is the adhesive bone filling agent of an embodiment of the present invention to be described later.

### <Method to use adhesive bone filling agent kit>

Next, the method to use the adhesive bone filling agent kit of an embodiment of the present invention will be described.

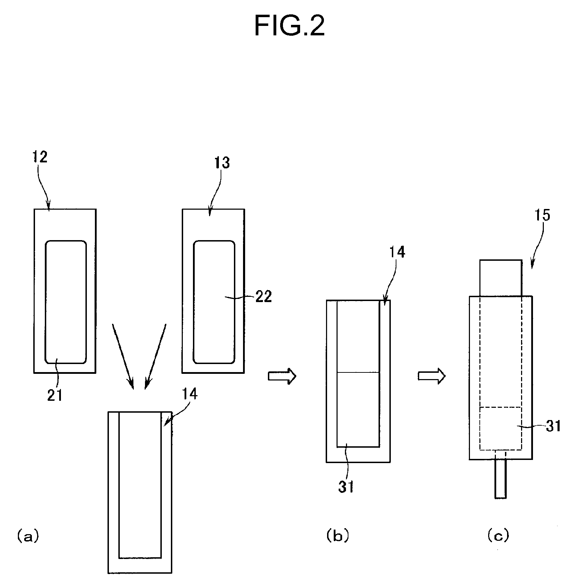

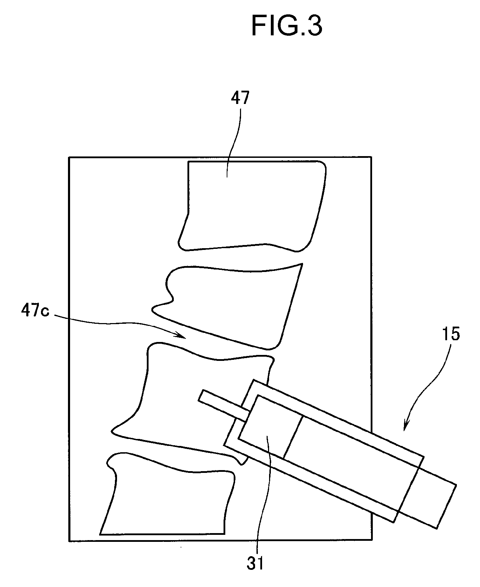

Figs. 2(a) to 2(c) and Fig. 3 are process views illustrating an example of a method to use an adhesive bone filling agent kit of an embodiment of the present invention, respectively.

First, as illustrated in Fig. 2(a), the first container 12 is opened to introduce the liquid component 21 into the third container 14, and the second container 13 is then opened to introduce the powder component 22 into the third container 14.

Next, as illustrated in Figs. 2(b) and 2(c), the liquid component 21 and the powder component 22 are thoroughly mixed in the third container 14 to form an adhesive bone filling agent 31, and the adhesive bone filling agent 31 is introduced into the injection container 15.

Next, a compression fracture part of a bone 47 is inflated with a balloon to form a hollow portion 47c at the bone tissue defective site and compression fracture site, and the adhesive bone filling agent 31 is injected into the hollow portion 47c from the injection container 15 as illustrated in Fig. 3.

It is possible to bond and fix the bone 47 by leaving to stand for a fixed period of time.

Incidentally, in the above process, it is preferable to introduce the powder component 22 into the liquid component 21 and to thoroughly mix them together. This makes it possible to simplify the mixing operation.

### <Adhesive bone filling agent>

First, the adhesive bone filling agent of an embodiment of the present invention will be described.

The adhesive bone filling agent 31 of an embodiment of the present invention is an adhesive bone filling agent that is injected into and coated on the bone tissue after mixing the liquid component 21 and the powder component 22 to adhere to the above bone tissue.

The liquid component 21 is a buffer solution comprising a water-soluble biocompatible polymer.

The water-soluble biocompatible polymer is a biopolymer or a synthetic polymer that is soluble in water and biocompatible.

It is preferable that the biopolymer be albumin or gelatin. This makes it possible to impart biocompatibility and absorbability to the adhesive bone cement produced.

It is preferable that the albumin be serum albumin of any one species or two or more species of cattle, swine, horse, and human or recombinant albumin. Specifically, examples of the albumin may include human serum albumin (HSA), and as the phosphate buffer solution containing a biopolymer, for example, a 42 w/v% phosphate buffer solution (pH 8.0) of human serum albumin (HSA) (hereinafter, abbreviated as 42 w/v% HSA) can be used.

It is preferable that the gelatin be gelatin of any one species or two or more species of cattle, swine, fish, and human or recombinant gelatin. Particularly, cod gelatin is preferable among fish gelatin since it is liquid at room temperature even in a high concentration. This makes it possible to impart cell adhesive property in addition to biocompatibility and absorbability to the adhesive bone filling agent produced.

In addition, it is preferable that the synthetic polymer be a polyether or water-soluble polymer having an amino group in a side chain or at a terminal.

It is preferable that the polyether having an amino group in a side chain or at a terminal be a poly(ethylene glycol) derivative having from 3 to 8 branches.

Examples of the poly(ethylene glycol) derivative having from 3 to 8 branches may include pentaerythritol tetra(poly(ethylene glycol)) ether, and examples of the poly(ethylene glycol) derivative having an amino group in a side chain or at a terminal may include tetraamine-terminated poly(ethylene glycol) (4 branches) (hereinafter, abbreviated as TAPEG). In addition, examples of the water-soluble polymer may include a polypeptide, and examples of the polypeptide having an amino group in a side chain or at a terminal may include polylysine.

The powder component 22 is a mixed powder of calcium phosphate as the first powder component and an organic acid-based crosslinking agent as the second powder component.

Calcium phosphate dissolves little by little and reconstitutes a low crystalline hydroxyapatite in the body to strength the bone.

Examples of calcium phosphate may include α-tricalcium phosphate (hereinafter, abbreviated as α-TCP (< 250 µm)) having a particle size of 250 µm or less. It is more favorable as the particle size is smaller. It is even more preferable as α-tricalcium phosphate is nanoparticles.

It is preferable that the calcium phosphate be one kind or a combination of two or more kinds of α-tricalcium phosphate (α-TCP), α'- tricalcium phosphate (α'-TCP), β-tricalcium phosphate (β-TCP), octa-calcium phosphate (OCP), dicalcium phosphate dibasic (DCPD), tetracalcium phosphate monoxide (TeCP), hydroxyapatite (HAp), and calcium monohydrogen phosphate. These calcium phosphates may be sintered or unsintered. This makes it possible to impart bone conductivity and osteoinductive capability.

In addition, it is preferable that the calcium phosphate be doped with a metal element in order to impart bone forming ability. As the metal element to be doped, for example, any one kind or two or more kinds of zinc, copper, strontium, silver, and titanium are preferable. This makes it possible to promote the bone formation by activation of the osteoblast.

In addition, it is preferable that the calcium phosphate be particles having a size of from 10 to 1000 nm. This makes it possible to increase the strength of the hardened product.

It is preferable that the organic acid-based crosslinking agent be one kind or a combination of two or more kinds of crosslinking agents obtained by modifying all the carboxyl groups of tartaric acid, malic acid, succinic acid, or citric acid with N-hydroxysuccinimide or N-hydroxysulfosuccinimide. This allows the active ester of the crosslinking agent to react with the amino group of the water-soluble biocompatible polymer contained in the liquid component to be hardened. Examples of the citric acid crosslinking agent may include trisuccinimidyl citrate (hereinafter, abbreviated as TSC) obtained by modifying the carboxyl group of citric acid with N-hydroxysuccinimide. In addition, examples of the malic acid crosslinking agent may include disuccinimidyl malate obtained by modifying two carboxyl groups of malic acid with N-hydroxysuccinimide. In addition, examples of the tartaric acid crosslinking agent may include disuccinimidyl tartrate obtained by modifying two carboxyl groups of tartaric acid with N-hydroxysuccinimide.

Furthermore, examples of the succinic acid crosslinking agent may include disuccinimidyl succinate obtained by modifying two carboxyl groups of succinic acid with N-hydroxysuccinimide.

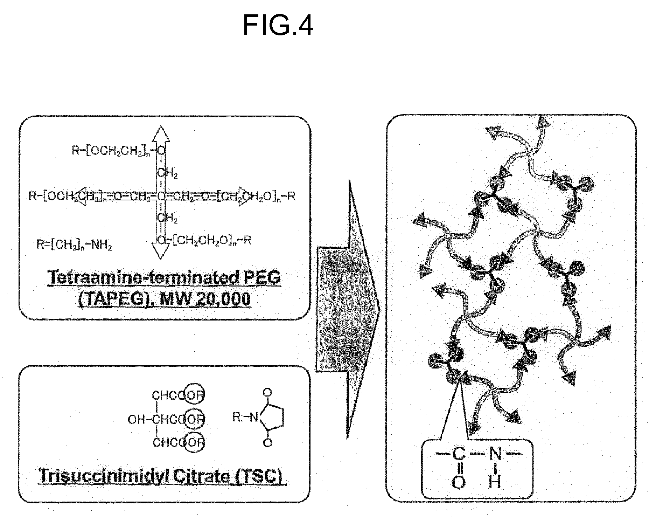

Fig. 4 is an explanatory view illustrating an example of the crosslinking reaction when TAPEG is mixed with trisuccinimidyl citrate (TSC) of an organic acid-based crosslinking agent. As illustrated in Fig. 4, the amino terminal of TAPEG is crosslinked along with the dissolution of TSC in the aqueous solution as the aqueous solution of TAPEG is mixed with TSC of the citric acid crosslinking agent that is one of the solid components, whereby a polymer gel is produced within from several minutes to several ten minutes.

It is preferable that an alkali component be added to the liquid component 21 or the powder component 22. This makes it possible to accelerate the adhering speed. Examples of the alkali component may include NaOH, NaHCO₃, and KOH.

An alkali solution is mixed with the liquid component 21 in the case of adding an alkali component to the liquid component 21. Examples of the alkali solution may include a 0.1 M NaOH solution (µL).

In addition, an alkali component formed into a powder from a substance dissolved in an alkali solution is mixed with the powder component 22 in the case of adding an alkali component to the powder component 22.

It is preferable that P/L, which is a ratio of calcium phosphate (P (g)) of the first powder component to the liquid component (L (g)), meet the condition of 1/1 < P/L < 3.5/1. This makes it possible to improve moldability. There is a risk of collapse in the case of 3.5/1 ≤ P/L. In addition, there is a risk of being nonuniform in the case of P/L ≤ 1/1.

The adhesive bone filling agent 31 of an embodiment of the present invention is an adhesive bone filling agent that adheres to the bone tissue and hardens for example by being injected into and coated on the bone tissue defective site and compression fracture site, and configured to contain a combination of the liquid component 21 composed of a buffer solution comprising a water-soluble biocompatible polymer and the powder component 22 comprising calcium phosphate and an organic acid-based crosslinking agent, and thus it is possible to increase the adhesive property to the bone tissue, to shorten the adhering time, and to increase the absorbability of the adhesive bone filling agent.

In addition, it is possible to set the bending strength of the adhesive bone filling agent to a moderate strength (1 MPa or more and 50 MPa or less). It is possible to greatly decrease the risk of fracture of the vertebral body bone adjacent to the surgical site by setting the bending strength to a moderate strength of 1 MPa or more and 50 MPa or less. The polymethyl methacrylate-based bone cement is too hard and thus concerned to cause further fracture of the peripheral vertebral body bone. On the other hand, the calcium phosphate-based bone filling agent is fragile and thus concerned to cause the fracture of the reinforced part. However, it is possible to decrease these risks by setting the strength thereof to a moderate strength, and thus a moderate strength is a great advantage in practical use.

The adhesive bone filling agent 31 of an embodiment of the present invention has a configuration in which the water-soluble biocompatible polymer is a biological polymers or synthetic polymer that is soluble in water and biocompatible, and thus it is possible to prepare a liquid component exhibiting high dispersibility in a buffer solution and to use the adhesive bone filling agent as an adhesive bone filling agent exhibiting high safety to a living body.

The adhesive bone filling agent 31 of an embodiment of the present invention has a configuration in which the biopolymer is albumin or gelatin, and thus it is possible to increase the absorbability of the adhesive bone filling agent.

The adhesive bone filling agent 31 of an embodiment of the present invention has a configuration in which the albumin is serum albumin of any one species or two or more species of cattle, swine, horse, and human or recombinant albumin, and thus it is possible to increase the absorbability and to enhance the cell adhesive property of the adhesive bone filling agent.

The adhesive bone filling agent 31 of an embodiment of the present invention has a configuration in which the gelatin is gelatin of any one species or two or more species of cattle, swine, fish, and human or recombinant gelatin, and thus it is possible to enhance the cell adhesive property in addition to the absorbability of the adhesive bone filling agent.

The adhesive bone filling agent 31 of an embodiment of the present invention has a configuration in which the synthetic polymer is a polyether or water-soluble polymer having an amino group in a side chain or at a terminal, and thus it is possible to enhance the strength of the bone filling agent itself by crosslinking the amino side chain or amino terminal of the synthetic polymer using an organic acid-based crosslinking agent.

The adhesive bone filling agent 31 of an embodiment of the present invention has a configuration in which the polyether having an amino group in a side chain or at a terminal is a poly(ethylene glycol) having from 3 to 8 branches, and thus it is possible to enhance the strength of the bone filling agent itself by crosslinking the amino side chain or the amino terminal using an organic acid-based crosslinking agent.

The adhesive bone filling agent 31 of an embodiment of the present invention has a configuration in which the poly(ethylene glycol) having from 3 to 8 branches is pentaerythritol tetra-poly(ethylene glycol) ether and the poly(ethylene glycol) having an amino group in a side chain or at a terminal is tetraamine-terminated poly(ethylene glycol) (4 branches), and thus it is possible to form a crosslinked structure by crosslinking the amine terminal of the polyether using an organic acid-based crosslinking agent and to enhance the strength of the bone filling agent itself.

The adhesive bone filling agent 31 of an embodiment of the present invention has a configuration in which the calcium phosphate is one kind or a combination of two or more kinds of α-tricalcium phosphate (α-TCP), α'- tricalcium phosphate (α'-TCP), β-tricalcium phosphate (β-TCP), octa-calcium phosphate (OCP), dicalcium phosphate dibasic (DCPD), tetracalcium phosphate monoxide (TeCP), hydroxyapatite (HAp), and calcium monohydrogen phosphate, and thus it is possible to increase the adhesive property thereof to the bone tissue.

The adhesive bone filling agent 31 of an embodiment of the present invention has a configuration in which the organic acid-based crosslinking agent is one kind or a combination of two or more kinds of crosslinking agents obtained by modifying all the carboxyl groups of tartaric acid, malic acid, succinic acid, or citric acid with N-hydroxysuccinimide or N-hydroxysulfosuccinimide, and thus it is possible to impart adhesive property to the bone filling agent.

The adhesive bone filling agent 31 of an embodiment of the present invention has a configuration in which an alkali component is added to the liquid component or the powder component, and thus it is possible to accelerate the adhering speed and to shorten the adhering time of the adhesive bone filling agent.

The adhesive bone filling agent 31 of an embodiment of the present invention has a configuration in which P/L, which is a ratio of the first powder component (P (g)) to the liquid component (L (g)),meets a condition of 1/1 < P/L < 3.5/1, and thus it is possible to increase the adhesive property to the bone tissue, to shorten the adhering time, and to increase the absorbability of the adhesive bone filling agent. In addition, it is possible to set the bending strength thereof to a moderate strength (1 MPa or more and 50 MPa or less).

The adhesive bone filling agent kit 11 of an embodiment of the present invention has a configuration which includes a first container 12 enclosing a liquid component 21, a second container 13 enclosing a powder component 22, a third container 14 for producing an adhesive bone filling agent 31 by mixing the powder component 22 with the liquid component 21, and an injection container 15 for injecting the adhesive bone filling agent 31 into a site to be adhered by the present adhesive agent and in which the liquid component is composed of a buffer solution comprising a water-soluble biocompatible polymer and the powder component is a mixed powder of calcium phosphate of the first powder component and an organic acid-based crosslinking agent of the second powder component, and thus it is possible to easily and quickly perform the adhesive treatment without requiring an instrument or a reagent other than this.

The adhesive bone filling agent and adhesive bone filling agent kit of embodiments of the present invention are not limited to the above embodiments but can be variously modified and implemented within the scope of the technical idea of the present invention. Specific examples of the present embodiments will be presented in the following examples. However, the present invention is not limited to these examples.

### Examples

### (Example 1)

### <Optimization test of solid / liquid (P/L)>

According to procedures as shown below, the forming ability of bone filling agent was investigated in various mixing ratios of solid / liquid (P/L), thereby finding an optimum condition of solid / liquid (P/L).

First, 42 w/v% HSA using human serum albumin (HSA, manufactured by Sigma-Aldrich Co., LLC.) or 30 w/v% cod gelatin was prepared as the liquid component (L), and α-TCP (manufactured by Wako Pure Chemical Industries, Ltd., those sieved to be < 250 µm) as calcium phosphate of the powder component (P) was prepared, and TSC as the organic acid-based crosslinking agent was prepared.

Next, five kinds of adhesive bone filling agents having P/L = 3.5 /1 (Test Examples 1 and 6), 3/1 (Test Examples 2 and 7), 2/1 (Test Examples 3 and 8), 1/1 (Test Examples 4 and 9), and 1/2 (Test Examples 5 and 10) were prepared by changing the mixing ratio (P/L) of α-TCP (< 250 µm) of the powder component (P) to 42 w/v% HSA or 30 w/v% cod gelatin of the liquid component (L). In the respective Test Examples, the amount of TSC added and the amount of the alkali (0.1 M NaOH solution (µL)) added were also changed as presented in Table 1.

The preparation conditions and the observation results of the moldability of the adhesive bone filling agents prepared using 42 w/v% HSA are presented in Table 1.

**[Table 1]**

| | Test Example 1 | Test Example 2 | Test Example 3 | Test Example 4 | Test Example 5 |
|---|---|---|---|---|---|
| P/L | 1/1 | 1.5/1 | 2/1 | 3/1 | 3.5/1 |
| α-TCP (g) | 2 | 2.4 | 2.66 | 3 | 3.11 |
| 42%HSA (g) | 2 | 1.6 | 1.33 | 1 | 0.89 |
| TSC (mg) | 159 | 127.2 | 105.7 | 79.5 | 70.8 |
| 0.1M NaOH sol. (µL) | 500 | 375 | 332.5 | 250 | 222.5 |
| Moldability | Non-uniform | Favorable | Favorable | Favorable | Fragile |

The preparation conditions and the observation results of the moldability of the adhesive bone filling agents prepared using 30 w/v% cod gelatin are presented in Table 2.

**[Table 2]**

| | Test Example 6 | Test Example 7 | Test Example 8 | Test Example 9 | Test Example 10 |
|---|---|---|---|---|---|
| P/L | 1/1 | 1.5/1 | 2/1 | 3/1 | 3.5/1 |
| α-TCP (g) | 2 | 2.4 | 2.66 | 3 | 3.11 |
| 30 % cod gelatin (g) | 2 | 1.6 | 1.33 | 1 | 0.89 |
| TSC (mg) | 159 | 127.2 | 105.7 | 79.5 | 70.8 |
| 0.1M NaOH sol. (µL) | 500 | 375 | 332.5 | 250 | 222.5 |
| Moldability | Fragile | Favorable | Favorable | Favorable | Favorable |

Next, five cylindrical molded samples having a diameter of 7 mm and a height of 14 mm were formed using these adhesive bone filling agents.

Next, the respective molded samples of the adhesive bone filling agents prepared using 42 w/v% HSA were left to stand for 24 hours in a humid environment at 37°C.

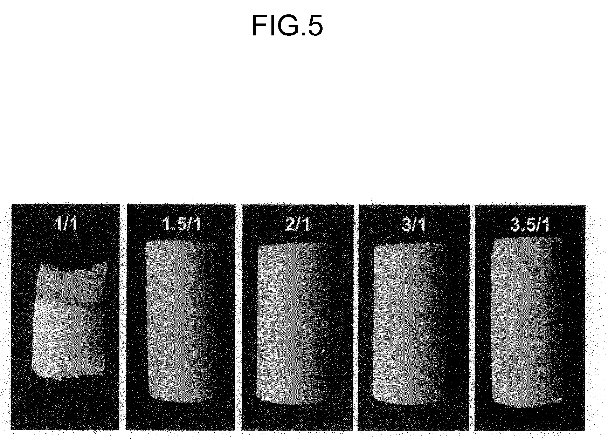

Fig. 5 is visual images (photographs) of the molded samples after leaving to stand for 24 hours in a wet environment at 37°C. As can be seen from the photographs, the moldability of the molded samples having P/L = 1.5/1 (Test Example 2), 2/1 (Test Example 3), and 3/1 (Test Example 4) was favorable even after leaving to stand.

On the other hand, the moldability of the molded sample having P/L = 1/1 (Test Example 1) was non-uniform after leaving to stand. This was because α-TCP had become non-uniform.

In addition, the molded sample having P/L = 3.5/1 (Test Example 5) was collapsed. This was because it was impossible to uniformly disperse α-TCP in the liquid and thus it was not able to keep the shape.

Consequently, it has been demonstrated that the condition of 1/1 < P/L < 3.5/1 is optimum in the case of 42 w/v% HSA.

Next, the respective molded samples were prepared using an adhesive bone filling agent prepared using 30 w/v% cod gelatin.

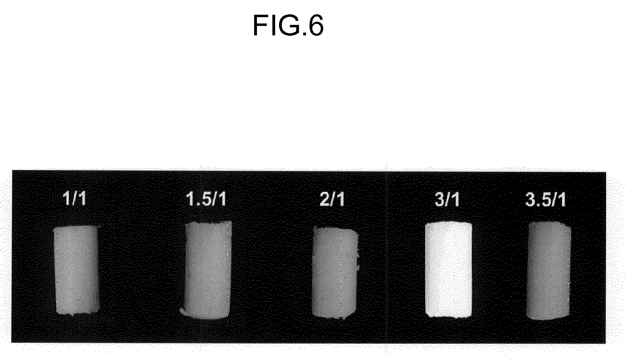

Fig. 6 is visual images (photographs) of molded samples after preparation. As can be seen from the photographs, the moldability of the molded samples having P/L = 1.5/1 (Test Example 7), 2/1 (Test Example 8), 3/1 (Test Example 9), and 3.5/1 (Test Example 10) was favorable.

On the other hand, the molded sample having P/L = 1/1 (Test Example 6) was fragile after leaving to stand. This was because the α-TCP content was not sufficient and thus the physical properties of cod gelatin was dominant.

Consequently, it has been demonstrated that the condition of 1/1 < P/L is optimum in the case of 30 w/v% cod gelatin.

### <Bone adhesive property test>

Next, the bone adhesive property test was conducted using the adhesive bone filling agent prepared using 42 w/v% HSA.

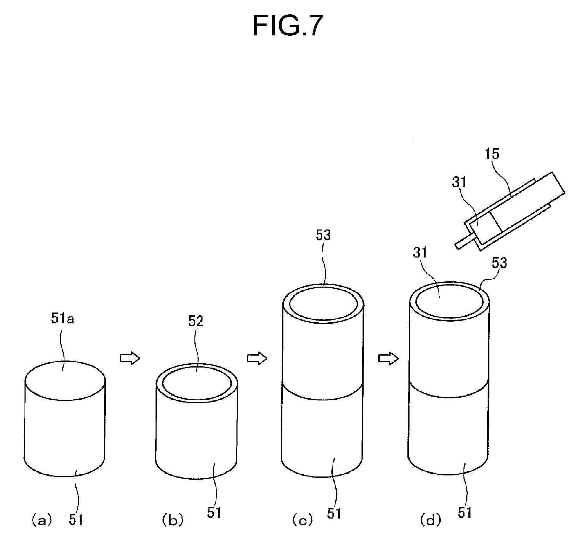

Figs. 7(a) to 7(d) are process views of the bone adhesive property test.

As a test piece 52 for the bone adhesive property test, a bone mimetic material of a living body was used.

The bone mimetic material of a living body was prepared by immersing ivory (diameter: 6 mm, thickness: 0.3 mm, disk-shaped) in a 25% aqueous solution of phosphoric acid for 180 seconds, then washing with a great amount of water, and drying.

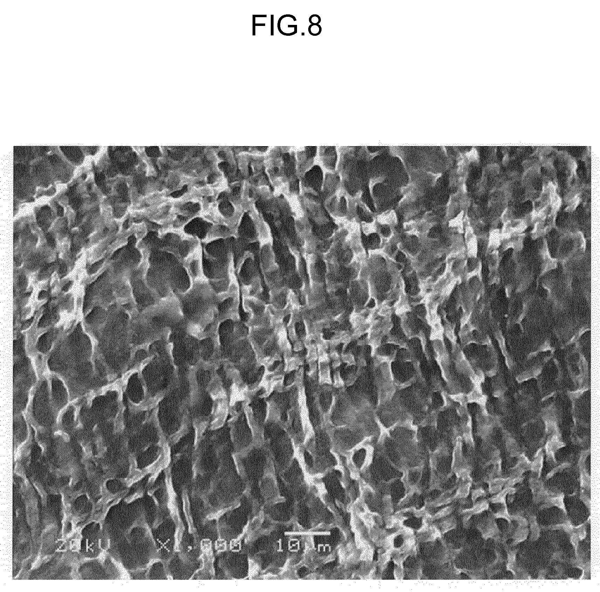

Fig. 8 is an electron micrograph illustrating an example of the surface of the bone mimetic material of a living body used in the present test.

### <Adhesion Test>

First, ivory (diameter: 6 mm, thickness: 0.3 mm, disk-shaped) was used as the test piece 52. The ivory was immersed in a 25% aqueous solution of phosphoric acid for 180 seconds, then washed with a great amount of water, and dried before the adhesion test, and then used. For the adhesion test, the test piece (ivory) was stuck on the surface 51 a of the cylindrical polymethyl methacrylate plastic rod 51.

Next, the resultant was covered with a cylindrical silicone 53 having an inner diameter of 7m and a height of 14 mm.

Next, the adhesive bone filling agent 31 was introduced into the cylinder of silicone 53. As the adhesive bone filling agent 31, an adhesive bone filling agent that was prepared using 42 w/v% HSA and had P/L = 3/1, a bone cement (commercially available product), and BioPex (manufactured by HOYA Corporation) were used.

Next, the adhesive strength of the adhesive bone filling agent and the like with respect to the test piece (ivory) 52 was examined using a texture analyzer.

The results of the adhesive strength of these adhesive bone filling agent and the like are presented in Table 3.

**[Table 3]**

| Kind of bone filling agent | Adhesive strength (MPa) |
|---|---|
| Material of present application (P/L = 3/1): Test Example 4 | 0.67 |
| Bone cement | 0.8 |
| BioPex | Not adhered |

The adhesive bone filling agent having P/L = 3/1 (Test Example 4) exhibited a higher adhesive strength as compared with BioPex. In addition, the bone cement exhibited a high adhesive strength value of 0.8 MPa but is not absorbable since it is a synthetic product, and thus the bone cement is not preferable.

### <Bending strength test>

First, a plate-shaped test piece having a length of 64 mm, a width of 10 mm, and a thickness of 4 mm was prepared.

Next, the bending strength of the molded sample was measured by autographing. As the control, commercially available bone cement and BioPex were used.

The results of the bending strength test are presented in Table 4.

**[Table 4]**

| | Bending strength (MPa) | |
|---|---|---|
| Material of present application (P/L = 3/1) Test Example 4 | 1.5 ± 0.3 | |
| Material of present application (P/L = 3/1) * | 11.3 ± 2.4 | * powder component (P) has a composition of α-TCP : TeCP : sodium monohydrogen phosphate : HAp = 75 : 18:5:2. |
| BioPex | 4.0 ± 0.3 | |
| Bone cement | 51.4 ± 3.6 | |

The adhesive bone filling agent having P/L = 3/1 (Test Example 4) had a lower bending strength as compared with BioPex and the bone cement, but the bending strength became higher than BioPex by changing the composition of the powder component.

### <Optimization test 2 of solid / liquid (P/L) (Test Examples A1 to A20: Run # A1 to A20)>

The forming ability of bone filling agent was investigated in various mixing ratios of solid / liquid (P/L) as follows.

First, TAPEG was dissolved in 0.1 M phosphate buffer solution (pH 6.0) to prepare a TAPEG (liquid component (L)).

Next, α-TCP, nanoapatite particles (spherical shape, average particle size: 40 nm, manufactured by SofSera Corporation), and TSC as the solid component (P) were weighed and thoroughly mixed together using a pencil stirrer. This mixed powder was added to the TAPEG solution dissolved in the 0.1 M phosphate buffer solution (pH 6.0) of the liquid component (L) and stirred and mixed using a pencil stirrer, thereby preparing an adhesive bone filling agent.

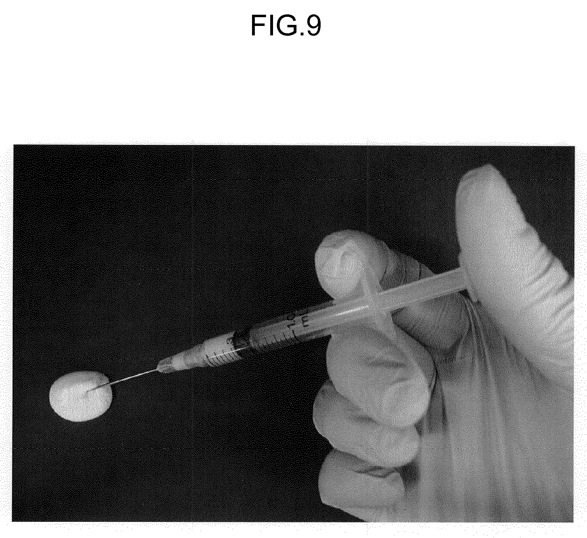

Fig. 9 is a photograph taken when the adhesive bone filling agent (Test Example A1) is discharged from a syringe.

Next, the adhesive bone filling agent was coated by the method previously described, and the bending strength thereof was measured.

The preparation conditions and measurement results of the bending strength of the adhesive bone filling agent thus prepared are presented in Table 5.

**[Table 5]**

| Run# | TAPEG conc. | TSC conc. | | HAp cont | *α*-TCP cont | | P/L ratio | Bending Strength |
|---|---|---|---|---|---|---|---|---|
| | mM | mM | mg | mg | mg | % | - | Mpe |
| A1 | 10 | 50 | 28.98 | 237.02 | 0.00 | 0 | 2/1 | 1.35 |
| A2 | 10 | 50 | 28.98 | 2133.90 | 237.10 | 10 | 2/1 | 1.43 |
| A3 | 10 | 50 | 28.98 | 1896.82 | 474.20 | 20 | 2/1 | 1.28 |
| A4 | 10 | 50 | 28.98 | 1422.61 | 948.41 | 40 | 2/1 | 3.01 |
| A5 | 10 | 50 | 28.98 | 474.20 | 1896.82 | 80 | 2/1 | 1.48 |
| A6 | 10 | 50 | 28.98 | 1782.61 | 1188.41 | 40 | 2.5/1 | 3.84 |
| A7 | 10 | 50 | 28.98 | 2142.62 | 1428.41 | 40 | 3/1 | 3.61 |
| A8 | 20 | 50 | 28.98 | 1782.61 | 1188.41 | 40 | 2.5/1 | 4.57 |
| A9 | 25 | 50 | 28.98 | 1782.61 | 1188.41 | 40 | 2.5/1 | 5.29 |
| A10 | 30 | 50 | 28.98 | 1782.61 | 1188.41 | 40 | 2.5/1 | 4.98 |
| A11 | 40 | 50 | 28.98 | 1782.61 | 1188.41 | 40 | 2.5/1 | 3.00 |
| A12 | 10 | 25 | 14.49 | 1791.30 | 1194.18 | 40 | 2.5/1 | 3.00 |
| A13 | 10 | 75 | 43.56 | 1773.84 | 1182.60 | 40 | 2.5/1 | 3.73 |
| A14 | 5 | 25 | 14.52 | 1791.30 | 1194.18 | 40 | 2.5/1 | 1.95 |
| A15 | 20 | 50 | 28.98 | 1485.51 | 1485.51 | 50 | 2.5/1 | 4.67 |
| A16 | 20 | 50 | 28.98 | 1188.40 | 1782.61 | 60 | 2.5/1 | 5.71 |
| A17 | 20 | 50 | 28.98 | 891.31 | 2079.71 | 70 | 2.5/1 | 5.82 |
| A18 | 25 | 50 | 28.98 | 891.31 | 2079.71 | 70 | 2.5/1 | 6.11 |
| A19 | 25 | 50 | 28.98 | 742.76 | 2228.27 | 75 | 2.5/1 | 5.48 |
| A20 | 25 | 50 | 28.98 | 594.20 | 2376.82 | 80 | 2.5/1 | 3.62 |

As presented in Table 5, it was possible to control the bending strength to 1.28 MPa or more and 6.11 MPa or less by controlling the P/L ratio, the TAPEG concentration and the TSC content.

### (Evaluation test 1 of adhesive bone filling agent)

### (Experiment)

NanoHAp: Nano-SHAp (spherical shape, average particle size: 40 nm) (manufactured by SofSera Corporation) was prepared as calcium phosphate of the powder material, and DST (disuccinimidyl tartrate) of a synthetic product was prepared as the organic acid-based crosslinking agent. Predetermined amounts of these DST and nanoHAp were weighed and thoroughly mixed together using a pencil stirrer, thereby obtaining a mixed powder. This mixed powder was added to the tube enclosing HSA (human serum albumin, Sigma-Aldrich Co., LLC., 0.1 M PBS pH 6.0) of the liquid component and stirred for 10 seconds using a pencil stirrer, thereby obtaining an adhesive bone filling agent. By this method, a plurality of adhesive bone filling agents were obtained by changing the mixing ratio of HSA, DST, and nanoHAp to one another.

Thereafter, this adhesive bone adhesive was introduced into a cylindrical (φ 7 mm, height: 14 mm) silicone tube and molded. The resultant was hardened by leaving to stand for 1 day at 37°C and taken out from the silicone mold to observe. At that time, the hardening time was qualitatively evaluated.

### (Result)

The results obtained by evaluating the state of the adhesive bone filling agent (uniformity and ease of injection) thus obtained and the state of the hardened product of the bone filling agent are presented in Table 6 (Test No.1 to 9), Table 7 (Test No. 10 to 18), and Table 8 (Test No. 19 to 27).

**[Table 6]**

| Test No. | 1 | 2 | 3 | 4 | 5 | 6 | 7 | 8 | 9 |
|---|---|---|---|---|---|---|---|---|---|
| DST (mmol) | 0.1 | 0.1 | 0.1 | 0.1 | 0.1 | 0.1 | 0.1 | 0.1 | 0.1 |
| P/L | 1/1 | 1/1 | 1/1 | 1.5/1 | 1.5/1 | 1.5/1 | 2/1 | 2/1 | 2/1 |
| HSA concentration (w/w%) | 30 | 35 | 40 | 30 | 35 | 40 | 30 | 35 | 40 |
| State of adhesive bone filling agent (uniformity/ ease of injection) | Uniform/ easy | Uniform/ easy | Uniform/ acceptable | Uniform/ easy | Uniform/ acceptable | Non-uniform/ acceptable | Uniform/ acceptable | Uniform/ difficult | Non-uniform/ difficult |
| Hardness of hardened product | Fragile | Fragile | Favorable | Fragile | Favorable | Favorable | Fragile | Favorable | Fragile |

**[Table 7]**

| Test No. | 10 | 11 | 12 | 13 | 14 | 15 | 16 | 17 | 18 |
|---|---|---|---|---|---|---|---|---|---|
| DST (mmol) | 0.2 | 0.2 | 0.2 | 0.2 | 0.2 | 0.2 | 0.2 | 0.2 | 0.2 |
| P/L | 1/1 | 1/1 | 1/1 | 1.5/1 | 1.5/1 | 1.5/1 | 2/1 | 2/1 | 2/1 |
| HSA concentration (w/w%) | 30 | 35 | 40 | 30 | 35 | 40 | 30 | 35 | 40 |
| State of adhesive bone filling agent (uniformity/ ease of injection) | Uniform/ easy | Uniform/ easy | Uniform/ difficult | Uniform/ easy | Uniform/ acceptable | Uniform/ difficult | Uniform/ easy | Uniform/ difficult | Non-uniform/ difficult |
| Hardness of hardened product | Fragile | Fragile | Fragile | Fragile | Favorable | Favorable | Fragile | Fragile | Fragile |

**[Table 8]**

| Test No. | 19 | 20 | 21 | 22 | 23 | 24 | 25 | 26 | 27 |
|---|---|---|---|---|---|---|---|---|---|
| DST (mmol) | 0.4 | 0.4 | 0.4 | 0.4 | 0.4 | 0.4 | 0.4 | 0.4 | 0.4 |
| P/L | 1/1 | 1/1 | 1/1 | 1.5/1 | 1.5/1 | 1.5/1 | 2/1 | 2/1 | 2/1 |
| HSA concentration (w/w%) | 30 | 35 | 40 | 30 | 35 | 40 | 30 | 35 | 40 |
| State of adhesive bone falling agent (uniformity/ ease of injection) | Uniform/ easy | Uniform/ easy | Uniform/ acceptable | Uniform/ easy | Uniform/ easy | Uniform/ difficult | Uniform/ easy | Uniform/ difficult | Non-uniform/ difficult |
| Hardness of hardened product | Favourable | Fragile | Fragile | Fragile | Favorable | Favorable | Fragile | Fragile | Fragile |

There was a tendency that the powder component containing HSA, DST, and nanoHAp was hardly uniformly mixed and the adhesive bone filling agent hardened faster in a case where the HSA concentration of the liquid component was high (40 w/w%). However, contrary to expectations, there was a tendency that the adhesive bone filling agent hardened faster and the hardness of the hardened product of the bone filling agent was also strong as the amount of DST was smaller. Among the conducted tests, Test No.5 was confirmed to exhibit excellent handling property of the adhesive bone filling agent, also to have a favorable hardness after 24 hours, and to be most balanced in terms of practicality.

### <Evaluation test 2 of adhesive bone filling agent>

### (Experiment)

The test was conducted under the same conditions as in the evaluation test 1 of the adhesive bone filling agent described above except that DSM (disuccinimidyl malate) of a synthetic product was used as the organic acid-based crosslinking agent instead of DST.

### (Result)

The results obtained by evaluating the state of the adhesive bone filling agent thus obtained and the state of the hardened product of the bone filling agent are presented in Table 9.

**[Table 9]**

| Test No. | 1 | 2 | 3 | 4 | 5 | 6 | 7 | 8 |
|---|---|---|---|---|---|---|---|---|
| DSM (mmol) | 0.1 | 0.1 | 0.1 | 0.1 | 0.1 | 0.2 | 0.2 | 0.2 |
| P/L | 1.5/1 | 1.5/1 | 1.5/1 | 2/1 | 2/1 | 1.5/1 | 1.5/1 | 2/1 |
| HSA concentration (w/w%) | 30 | 35 | 40 | 35 | 40 | 35 | 40 | 35 |
| State of adhesive bone filling agent (uniformity/ ease of injection) | Uniform/ easy | Uniform/ acceptable | Non-uniform / acceptable | Non-uniform / acceptable | Non-uniform / difficult | Uniform/ acceptable | Non-uniform / acceptable | Uniform / difficult |
| Hardness of hardened product | Fragile | Fragile | Favorable | Favorable | Fragile | Fragile | Fragile | Slightly fragile |
| Test No. | 9 | 10 | 11 | 12 | | | | |
| DSM(mmol) | 0.4 | 0.4 | 0.4 | 0.4 | | | | |
| P/L | 1.5/1 | 1.5/1 | 1.5/1 | 2/1 | | | | |
| HSA concentration (w/w%) | 30 | 35 | 40 | 35 | | | | |
| State of adhesive bone filling agent (uniformity/ ease of injection) | Uniform/ acceptable | Non-uniform / acceptable | Uniform/ easy | Uniform/ difficult | | | | |
| Hardness of hardened product | Fragile | Slightly fragile | Fragile | Fragile | | | | |

The adhesive bone filling agent using DSM had a longer hardening time and was easily molded as compared with the adhesive bone filling agent using DST. There was a tendency that the viscosity of this adhesive bone filling agent was lower as compared with the adhesive bone filling agent using the same amount of DST. It has been demonstrated that Test No.3 and Test No.4 are balanced from the handling property of the adhesive bone filling agent and the hardness of the hardened product of the bone filling agent after 24 hours.

Hence, it has been demonstrated that DSM of a highly hydrophobic organic acid-based crosslinking agent is more useful than DST as the powder component of the adhesive bone filling agent. From this result, it has been demonstrated that the handling property of the adhesive bone filling agent and the hardness of the hardened product of the bone filling agent are excellent in the case of using TSC of a highly hydrophobic organic acid-based crosslinking agent as the powder component of the adhesive bone filling agent as well.

<Measurement of mechanical strength of HSA bone filling agent>

### (Experiment)

In the test piece for the bending test, DSM and nanoHAp were used as the powder material (P) and 35 w/w% HSA (0.1 M PBS, pH 6) was used as the liquid material (L), and DSM was weighed so as to have the P/L ratio of 2/1 and to be 125 mM and mixed with nanoHAp weighed, and the mixture was then thoroughly mixed using a pencil stirrer, thereby obtaining a mixed powder. This mixed powder was added to the HSA solution and then stirred using a pencil stirrer to prepare an adhesive bone filling agent, and this adhesive bone filling agent was filled in a silicone mold having dimensions of 40 x 10 x 4 mm and interposed between glass plates to mold. The resultant was hardened by leaving to stand for 1 day at 37°C to obtain the hardened product of the bone filling agent, and this hardened product of the bone filling agent was subjected to the bending test.

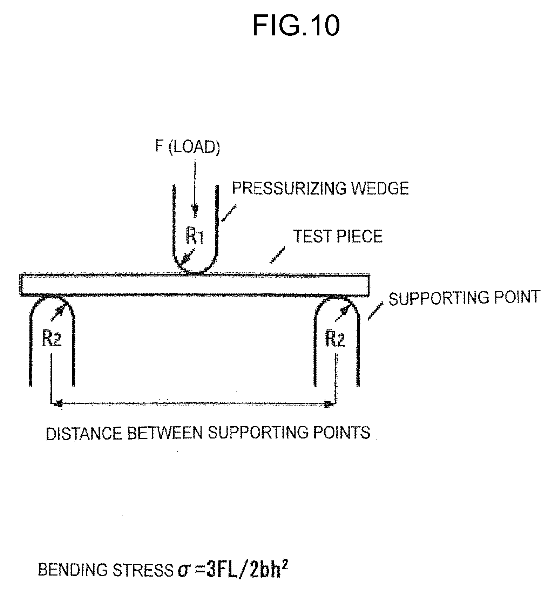

The outline of the bending test is illustrated in Fig. 10. For the bending test, Shimadzu Autograph AGS-H was used, and the evaluation was performed using a load cell of 1 KN. As illustrated in Fig. 10, the test piece was supported between the supporting points having a constant radius of curvature R, a load was applied to the middle therebetween using a pressurizing wedge, the load when the test piece was broken was adopted as the strength.

### (Result)

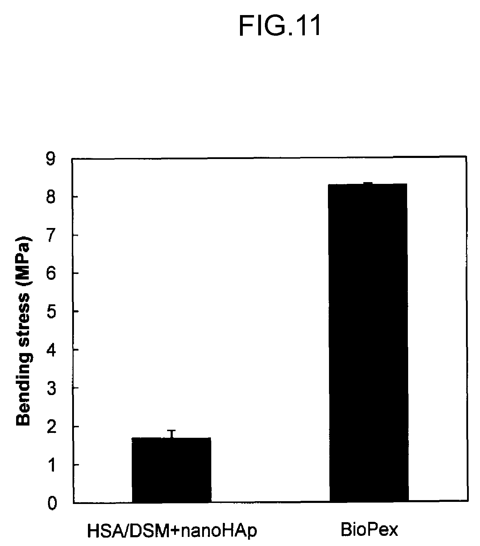

The results are illustrated in Fig. 11. The bending stress of the hardened product of this bone filling agent using HSA was 1.7 MPa (±0.2). Meanwhile, the strength of BioPex (manufactured by HOYA Corporation) of a commercially available bone filling agent was 4 times or more the strength of this bone filling agent, and thus it has been considered that the investigation on other biocompatible polymers is required in order to achieve an improvement in bending stress.

### <Measurement of mechanical strength of TAPEG bone filling agent>

### (Experiment)

In the test piece for the bending test, TSC (trisuccinimidyl citrate), nanoHAp, and α-TCP (α-tricalcium phosphate) which were synthetic products were used as the powder material (P) and TAPEG/ 0.1 M PBS (pH 6) (TAPEG: tetraamine terminated poly(ethylene glycol) (MW: 20,000) (manufactured by NOF CORPORATION) was used as the liquid material (L).

For the powder material, the proportions of α-TCP and nanoHAp were determined excluding the mass of TSC. The method for preparing the test piece was as follows. TSC, nanoHAp, and α-TCP were weighed and thoroughly mixed together using a pencil stirrer. This mixed powder was added to the TAPEG solution and stirred using a pencil stirrer to prepare an adhesive bone filling agent, this adhesive bone filling agent was filled in the silicone tube having dimensions of 40 x 10 x 4 mm and interposed between glass plates to mold. The resultant was hardened by leaving to stand for 1 day at 37°C to obtain the hardened product of the bone filling agent, and this hardened product of the bone filling agent was subjected to the bending test.

### (Result -1) Evaluation on effect of TAPEG concentration on bending strength

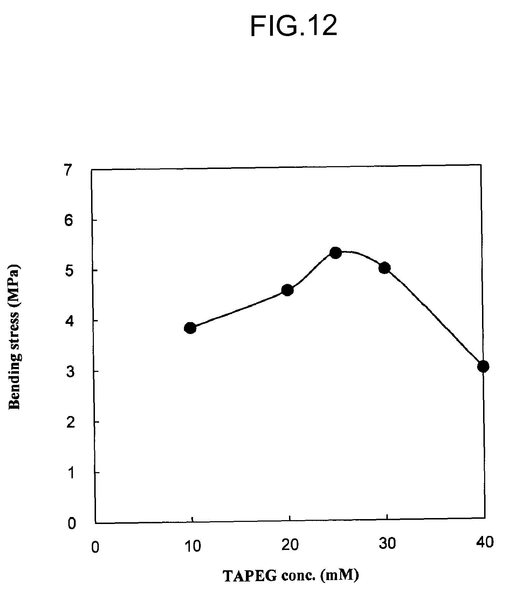

The TAPEG dependence of the bending strength was evaluated by setting the P/L ratio to 2.5/1 and the mass of α-TCP to be 40% of that of the powder material excluding TSC. As illustrated in Fig. 12, it has been confirmed that the bending stress is the highest when the TAPEG is 25 mM.

### (Result -2) Evaluation on effect of TSC concentration on bending strength

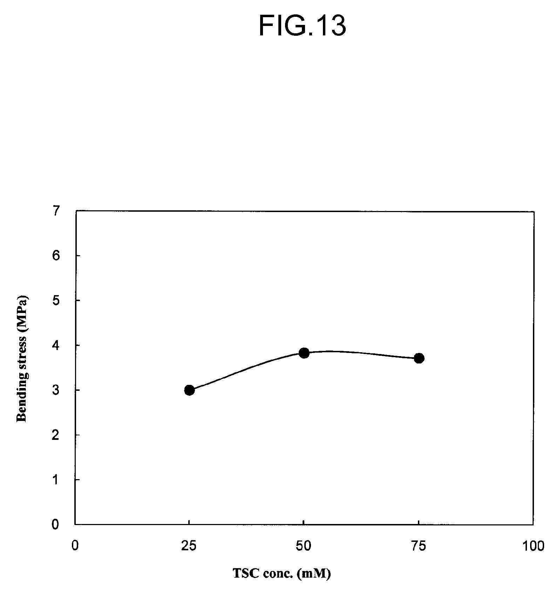

The TSC concentration dependence of the bending strength was evaluated by setting the P/L ratio to 2.5/1 and the mass of α-TCP to be 40% of that of the powder material excluding TSC. As illustrated in Fig. 13, it has been confirmed that the bending stress is the highest when the TSC concentration is 50 mM.

### (Result -3) Evaluation on effect of P/L ratio on bending strength

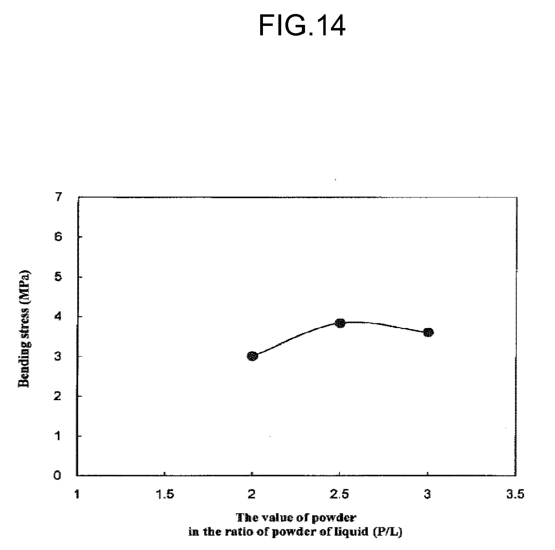

The TSC concentration dependence of the bending strength was evaluated by setting the mass of α-TCP to be 40% of that of the powder material excluding TSC. As illustrated in Fig. 14, it has been confirmed that the bending stress is the highest when the P/L ratio is 2.5/1. In the preliminary experiment, it was impossible to mold at P3.5/L1 since the powder was too much.

### (Result -4) Evaluation on effect of α-TCP concentration on bending strength

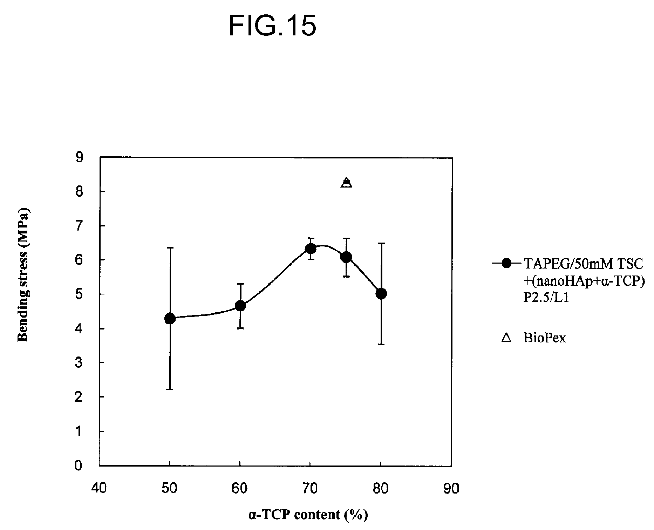

The TSC concentration dependence of the bending strength was evaluated by fixing the P/L ratio to 2.5/1 and setting the mass of α-TCP to be 50%, 60%, 70%, 75%, and 80% of that of the powder material excluding TSC. The bending stress of BioPex having an α-TCP content of 75% was also measured under the same conditions. As illustrated in Fig. 15, it has been confirmed that the bending stress is the highest and the reproducibility is also high when the content of α-TCP is 70%.

### (Result -5) Condition optimization of component of bone filling agent affecting bending strength

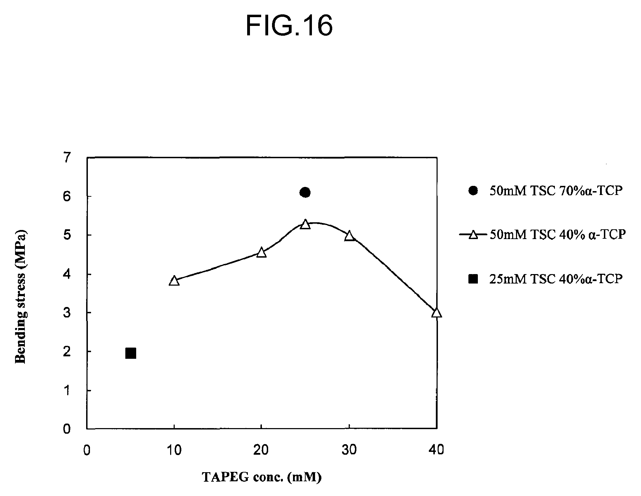

It was indicated that the bending stress was the highest at 25 mM TAPEG/ 50 mM TSC + (nanoHAp + 70% α-TCP) and the P/L ratio of 2.5/1, and thus the bending test was conducted under this condition. The P/L ratio was set to 2.5/1. As illustrated in Fig. 16, it has been confirmed that the bending stress is the highest at 25 mM TAPEG/ 50 mM TSC + (nanoHAp + 70% α-TCP) and the Powder/Liquid ratio of 2.5/1.

### Industrial Applicability

The adhesive bone filling agent and adhesive bone filling agent kit of the present invention relate to an adhesive bone filling agent which is highly adhesive to the bone tissue, adheres in a short period of time, and is highly absorbable and an adhesive bone filling agent kit, and this adhesive bone filling agent can be used in the treatment of compression fracture since it adheres in a shorter period of time, has properties of a higher strength and higher adhesive property, and is more highly absorbable than a bone cement and a bone paste, and thus it is usable in the industry of medical devices, materials, and equipment.

### Reference Signs List

- 11: Adhesive bone filling agent kit
- 12: First container
- 13: Second container
- 14: Third container
- 15: Injection container
- 21: Liquid component
- 22: Powder component
- 23: X-ray detector
- 24: Pedestal
- 31, 32: Adhesive bone filling agent
- 47: Bone
- 47c: Hollow portion
- 51: Polymethyl methacrylate plastic rod
- 51a: Surface
- 52: Test piece
- 53: Cylindrical silicone

## Claims

1. An adhesive bone filling agent which adheres to bone tissue and hardens, the adhesive bone filling agent comprising:
a combination of a liquid component composed of a buffer solution comprising a water-soluble biocompatible polymer and a powder component comprising calcium phosphate and an organic acid-based crosslinking agent.

2. The adhesive bone filling agent according to claim 1, wherein the water-soluble biocompatible polymer is a biopolymer or a synthetic polymer.

3. The adhesive bone filling agent according to claim 2, wherein the biopolymer is albumin or gelatin.

4. The adhesive bone filling agent according to claim 3, wherein the albumin is serum albumin of any one species or two or more species of cattle, swine, horse, and human or recombinant albumin.

5. The adhesive bone filling agent according to claim 3, wherein the gelatin is gelatin of any one species or two or more species of cattle, swine, fish, and human or recombinant gelatin.

6. The adhesive bone filling agent according to claim 2, wherein the synthetic polymer is a polyether or water-soluble polymer having an amino group in a side chain or at a terminal.

7. The adhesive bone filling agent according to claim 6, wherein the polyether is a poly(ethylene glycol) derivative having from 3 to 8 branches.

8. The adhesive bone filling agent according to claim 7, wherein the poly(ethylene glycol) having from 3 to 8 branches is pentaerythritol tetra(poly(ethylene glycol)) ether.

9. The adhesive bone filling agent according to claim 6, wherein the water-soluble polymer is a polypeptide.

10. The adhesive bone filling agent according to claim 9, wherein the polypeptide is polylysine.

11. The adhesive bone filling agent according to any one of claims 1 to 10, wherein the calcium phosphate is one kind or a combination of two or more kinds of α-tricalcium phosphate (α-TCP), α'- tricalcium phosphate (α'-TCP), β-tricalcium phosphate (β-TCP), octa-calcium phosphate (OCP), dicalcium phosphate dibasic (DCPD), tetracalcium phosphate monoxide (TeCP), hydroxyapatite (HAp), and calcium monohydrogen phosphate.

12. The adhesive bone filling agent according to any one of claims 1 to 11, wherein the calcium phosphate is doped with a metal element.

13. The adhesive bone filling agent according to claim 12, wherein the metal element is one kind or two or more kinds of zinc, copper, strontium, silver, and titanium.

14. The adhesive bone filling agent according to any one of claims 1 to 13, wherein the calcium phosphate is in the form of particle having a size of from 10 to 1000 nm.

15. The adhesive bone filling agent according to any one of claims 1 to 14, wherein the organic acid-based crosslinking agent is one kind or a combination of two or more kinds of crosslinking agents obtained by modifying all the carboxyl groups of tartaric acid, malic acid, succinic acid, or citric acid with N-hydroxysuccinimide or N-hydroxysulfosuccinimide.

16. The adhesive bone filling agent according to any one of claims 1 to 15, wherein an alkali component is added to the liquid component or the powder component.

17. The adhesive bone filling agent according to any one of claims 1 to 16, wherein P/L, which is a ratio of the calcium phosphate (P (g)) to the liquid component (L (g)), meets a condition of 1/1 < P/L < 3.5/1.

18. An adhesive bone filling agent kit comprising:
a first container enclosing a liquid component composed of a buffer solution comprising a water-soluble biocompatible polymer;
a second container enclosing a powder component comprising calcium phosphate and an organic acid-based crosslinking agent;
a third container for producing an adhesive bone filling agent by mixing the powder component with the liquid component; and
an injection container for injecting the adhesive bone filling agent into a site to be adhered.
